# EUROPEAN PATENT SPECIFICATION

(11) **EP 2 283 889 B1**
(45) Date of publication and mention of the grant of the patent: **26.12.2012**
(21) Application number: 10006887.3
(22) Date of filing: 02.07.2010
(51) Int. Cl.: A61M 16/10, A61M 16/16, B01D 53/26, A61M 16/08

(54) **Humidification cartridge**
Befeuchtungskartusche
Cartouche d'humidification

(30) Priority: 12.08.2009 IT BO20090544
(43) Date of publication of application: 16.02.2011
(73) Proprietor: Covidien AG, 8212 Neuhausen am Rheinfall (CH)
(72) Inventor: Borali, Stefano, 41037 Mirandola (MO) (IT)
(74) Representative: Olsson, Carl H.S.

(56) References cited:
- WO-A1-99/27988
- WO-A1-2004/112873
- WO-A2-90/04425
- US-A- 3 874 379
- US-A- 3 916 891
- US-A- 4 319 566
- US-A- 4 417 574
- US-A1- 2006 037 613

## Description

The present invention relates to a humidification cartridge to be used in an active humidification system for respiratory care. Furthermore, the present invention relates to a patient breathing system, in detail for the humidification/dehumidification of an inhalation/exhalation gas flowing in a ventilation circuit of a patient.

In clinical praxis when a patient undergoes to invasive mechanical ventilation meaning that upper airways are bypassed due to tracheal intubation, it is necessary to prevent loss of patient airways heat and humidity through passive devices (Heat and Moisture Exchangers) or active humidification systems. Active humidifiers enables delivery of a proper amount of heat and humidity to the patient inspiratory gases, thus reducing the impact of the dry and cold inspiratory air flow on the respiratory tract mucosae of the patient.

Usually, the air delivered to the patient is humidified during its passage through a heating device comprising a water reservoir (humidification cartridge). Moreover, in order to convey to the patient a gas with a physiological temperature, and furthermore to avoid the condensation of humidity enriched gases in the inspiratory limb, electrical resistances are used to heat at least a portion of said limb, so that the temperature inside said limbs is maintained at controlled levels preventing any fall below the dew point of the passing gas mixture. Obviously, all this involves a remarkable energy consumption.

A further drawback shown by the traditional systems is represented by the fact that the gases exhaled by the patient, and also containing water vapour, are then sent to the ventilator where the condensation of said vapour can take place with relative malfunctioning and/or damages to the ventilator. For instance, the flow sensors present in the ventilator can be damaged by the formation of condensed water.

Therefore, systems to trap the progressively formed condensed water in order to avoid that said condensed water enters the ventilators are known in the art. The condensed water is then kept in particular trapping devices placed in the circuit expiratory limb. However, these trapping devices of the condensed water must be periodically emptied when the level of condensed water reaches a predefined limit. Obviously, this requires a continuous overseeing of the system by the sanitary operators.

Furthermore, the condensed water trapped in the trapping devices is directly discharged in the environment, namely before having undergone any filtration step. It is clear that this involves the danger of transmission of pathogenous germs in the environment.

US 3,916,891 describes a device for moistening respiratory air having a hollow space. Water to be evaporated is discharged and evaporated via a heater in the hollow space. Condensed water in the hollow space is guided into a collector via a nozzle comprising a dirt filter and finally discharged.

Therefore, the aim of the present invention is to produce a humidification cartridge to be used in an active humidification system being part of a patient breathing system which is free from the aforesaid drawbacks and, at the same time, can be easy and inexpensive to produce.

According to the present invention a humidification cartridge is provided according to what claimed in Claim 1, or in any one of the claims directly or indirectly depending on Claim 1.

For a better understanding of the present invention, it is now described a preferred embodiment, merely as a non limitative example and with a reference to the enclosed drawings, wherein:

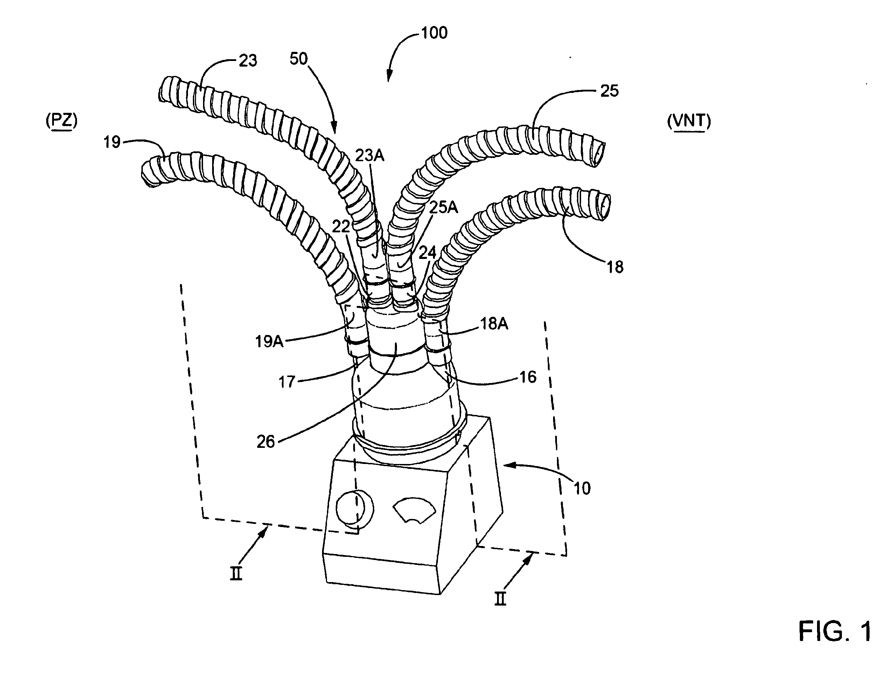

- figure 1 illustrates a perspective view of a humidification cartridge according to the present invention;
- figure 2 illustrates a longitudinal section A-A (taken along a plane II - see figure 1) of the humidification cartridge shown in figure 1;
- figure 3 illustrates an exploded view of the components of the humidification device as shown in figures 1, 2.

In the enclosed figures, the reference number 10

indicates, as a whole, a humidification cartridge to be used in an active humidification system part of a breathing system 100 for a patient (PZ).

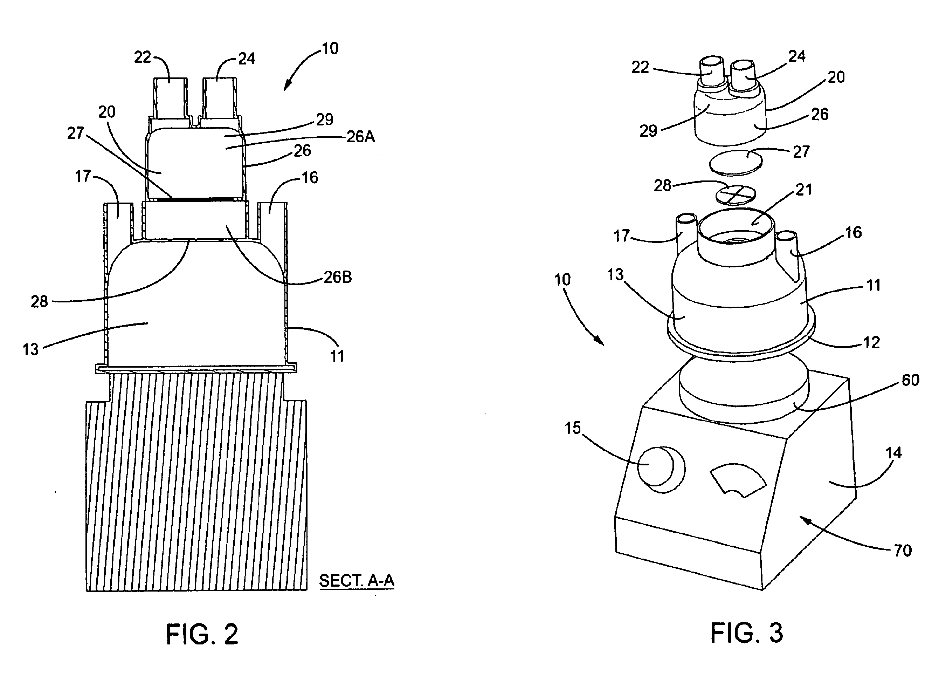

The humidification cartridge 10 substantially comprises a container 11 formed by a plastic cap and a base consisting of a heat conducting plate 12 (figure 3). The container 11 and the heat conducting plate 12 define a containing and heating chamber 13. Water present in the heating chamber 13 is evaporated because the heat conducting plate 12 is heated by contact with a heating plate 60 of a humidifier 70. The heating plate 60 is heated, in its turn, by means of a plurality of electrical resistances of a known kind and which are not shown.

The humidifier 70 can be of various types and models.

In particular, the humidifier 70 comprises a carter 14 containing its functional elements, such as electronic circuits controlling/setting the electric resistances in the heating plate 60, at least a thermostat, etc. Manually regulating devices 15 of a known type are further provided on the carter 14, by means of which an operator can pre-set the heating cycle of the water contained in the chamber 13.

The cap container 11 foresees an inlet opening 16 and an outlet opening 17 for respiratory gases which are sent by a ventilator (VNT) to the patient (PZ) using the ventilation circuit 100. In particular, an end 18A of a first inhalation duct 18 is inserted in the inlet opening 16, said duct 18 being able to transport respiratory gases from the ventilator (VNT) to the container 11. Analogously, an end 19A of a second inhalation duct 19 is housed in the outlet opening 17, through which the respiratory gases flow from the container 11 to patient (PZ). Since the gas must reach the patient (PZ) with a controlled temperature and it must be avoided that the humidity contained in the ventilation gases condenses in the second inhalation duct 19, said second inhalation duct 19 is advantageously heated by at least one electric resistance (not shown).

A very important element of the present invention is represented by a condensed water collector 20 housed in a respective opening 21, also formed in the container 11. In other words, an important element of the present invention consists in that the condensed water collector 20 is integrated in the humidification cartridge 10, and in particular in the container 11 belonging to said humidification cartridge 10.

The condensed water collector 20 is provided with a first inlet connector 22 on which an end 23A of a first exhalation duct 23 is fitted, and with a second outlet connector 24 to which an end 25A of a second exhalation duct 25 is attached.

The ducts 18, 19, 23, 25 form as a whole a ventilation circuit 50 from and to patient (PZ). The condensed water collector 20 further comprises a main cylindrical body 26, divided in its height by a microporous filtering septum 27, and closed at the bottom by a one-way valve 28.

In another illustration of background art, if the microporous filtering septum 27 is able to provide a hindrance to the gas flowing through heating chamber 13, the one-way valve 28 is absolutely optional and thus it can be omitted.

The main body 26 is separated by the microporous filtering septum 27 in an upper space 26A, defined by a head wall 29 and by the microporous filtering septum 27, and in a lower space 26B defined, on its turn, by the microporous filtering septum 27 and by the one-way valve 28.

The functioning of the ventilation system 100, and in particular of its humidification cartridge 10, is the following:
[A] the gas (generally air or oxygen more or less mixed with other gases) coming from the ventilator (VNT) passes through the duct 18 and is humidified passing in the chamber 13, wherein it is mixed with the water vapour created by heating the water contained in the chamber 13 by means of the heat conducting plate 12, which acts as the bottom of the container 11;
[B] the mixture gas/water vapour is sent to the patient (PZ) by using the duct 19 thanks to the thrust always exerted by the ventilator (VNT); the condensation of the water vapour contained in the mixture does not occur in the duct 19, because the duct 19, on its turn, is heated by at least an electric resistance;
[C] on their turn, the gases exhaled by the patient (PZ) are sent to condensed water collector 20 through duct 23; the condensation of water vapour contained in the exhalation gases occurs in the condensed water collector 20;
[E] the particles of condensed water are then slowly micro-filtrated passing through the microporous filtering septum 27 before being stored in the lower space 26B until the weight of the water collected in said space 26B opens the one-way valve 28;
[D] the condensed water is then periodically and automatically, namely without any intervention of an external operator, discharged from the lower space 26B to the container 11.

Therefore, the condensed water coming from the gases exhaled by the patient is not collected in a separated condensed water collector (condensed water trap), which can be placed anywhere in the ventilation circuit, and which must be periodically emptied, but is directly collected in the humidification cartridge after having undergone a suitable filtration to avoid that undesired elements coming from the patient are put into circulation.

The microporous filtering septum 27 also acts as a protection barrier for the one-way valve 28. It has been experimentally verified that the warm part formed by the container 11 and by the heat conducting plate 12 does not hinder the condensation of water vapour in the condensed water collector 20 because the water contained in the chamber 13 is heated at a temperature slightly higher than 40°C. Therefore, the humidified air lapping the base of the condensed water collector 20 is not hot enough to hinder the condensation of the water vapour.

Furthermore, the exhalation circuit starting from the patient (PZ) and leading into the condensed water collector 20 is not thermally regulated, because in this case the aim is to remove the water contained in the exhaled gas so that said water does not condense inside the ventilator (VNT), thus damaging the internal humidity sensitive electronic components.

One of the particular aims of the present invention is the removal, by means of condensation, of a larger amount of water from the exhalation flow. By integrating the condensed water collector 20 in the humidification cartridge 10 its positioning is always lower than the patient (PZ), so that the condensed water drops, thanks to gravity, in the condensed water collector 20. Moreover, the instructions for use recommend to always check the correct mounting of the circuit in order to avoid liquid-collecting loops.

The main advantages of the ventilation system and of the humidification cartridge objects of the present invention are the following:
(a) no further need to periodically empty the trapping collectors of condensed water, because this water is directly discharged in the humidification cartridge;
(b) no further dispersion of pathogen germs in the environment;
(c) filtering the condensed water before using it again in the circuit for avoiding dispersion of pathogen germs in the cartridge with consequent bacteria growth; and
(d) moderate reduction of energy consumption for heating the humidification water of the ventilation circuit because the humidification cartridge is continuously fed with lukewarm condensed water coming from the exhalation duct.

## Claims

1. Humidification cartridge (10) comprising:
a containing and heating chamber (13) of a liquid able to evaporate; and
a heater (12) for heating the liquid contained in said chamber (13);
said cartridge (10) comprises a condensed water collector (20) integrated in said chamber (13), **characterized in that** said condensed water collector (20) comprises a main body (26), divided in height by a microporous filtering septum (27) and closed on the bottom by a one-way valve (28).

2. Humidification cartridge (10) according to Claim 1, **characterized in that** on the wall of said chamber (13) an inlet opening (16) and an outlet opening (17), respectively, of a first inhalation duct (18) and of a second inhalation duct (19), and an opening (21) to house said condensed water collector (20) are provided.

3. Humidification cartridge (10) according to one of the claims 1 or 2, **characterized in that** the main body (26) is divided by the microporous filtering septum (27) in an upper space (26A), defined by a head wall (29) and by the microporous filtering septum (27), and a lower space (26B) defined, on its turn, by the microporous filtering septum (27) and by the valve (28).

4. Humidification cartridge (10) according to Claim 3, **characterized in that** said condensed water collector (20) comprises a first inlet connector (22) on which an end (23A) of a first exhalation duct (23) is fitted, and a second outlet connector (24) to which an end (25A) of a second exhalation duct (25) is attached.

5. Humidification cartridge (10) according to Claim 1, **characterized in that** said heater (12) is heated by contact with a heating plate (60) of a humidifier (70), the heating plate (60) being heated, in its turn, by means of a plurality of electrical resistances.

6. Humidification cartridge (10) according to Claim 1, **characterized in that** said humidifier (70) comprises a carter (14) containing its functional elements, such as electronic circuits controlling/setting the electric resistances in the heating plate (60), and at least a thermostat.

7. Humidification cartridge (10) according to Claim 6, **characterized in that** said humidifier (70) further comprises manually regulating devices (15) by means of which an operator can pre-set the heating cycle of the water contained in said chamber (13).

8. Ventilation system (100) for a patient (PZ); said system (100) comprising
- a ventilator (VNT);
- a ventilation circuit (50) of at least a ventilation gas from and to the patient (PZ);
**characterized by** at least a humidification cartridge (10) according to any of the claims 1 to 7, wherein said humidification cartridge (10) and a condensed water collector (20) are mutually physically integrated.

## Patentansprüche

1. Befeuchtungspatrone (10), die Folgendes umfasst:
eine Aufnahme- und Heizkammer (13) für eine Flüssigkeit, die verdampfen kann; und
eine Heizeinrichtung (12) zum Erhitzen der in der Kammer (13) enthaltenen Flüssigkeit;
wobei die Patrone (10) einen Sammler (20) für kondensiertes Wasser, der in die Kammer (13) integriert ist, umfasst, **dadurch gekennzeichnet, dass** der Sammler (20) für kondensiertes Wasser einen Hauptkörper (26) umfasst, der in Höhenrichtung durch eine mikroporöse Filterwand (27) unterteilt ist und an der Unterseite durch ein Einwegventil (28) verschlossen ist.

2. Befeuchtungspatrone (10) nach Anspruch 1, **dadurch gekennzeichnet, dass** in der Wand der Kammer (13) eine Einlassöffnung (16) und eine Auslassöffnung (17) eines ersten Einatmungsrohrstutzens (18) bzw. eines zweiten Einatmungsrohrstutzens (19) und eine Öffnung (21) für die Unterbringung des Sammlers (20) für kondensiertes Wasser vorgesehen sind.

3. Befeuchtungspatrone (10) nach einem der Ansprüche 1 oder 2, **dadurch gekennzeichnet, dass** der Hauptkörper (26) durch die mikroporöse Filterwand (27) in einen oberen Raum (26A), der durch eine Kopfwand (29) und durch die mikroporöse Filterwand (27) definiert ist, und einen unteren Raum (26B), der seinerseits durch die mikroporöse Filterwand (27) und durch das Ventil (28) definiert ist, unterteilt ist.

4. Befeuchtungspatrone (10) nach Anspruch 3, **dadurch gekennzeichnet, dass** der Sammler (20) für kondensiertes Wasser einen ersten Einlassverbinder (22), an dem ein Ende (23A) eines ersten Ausatmungsrohrstutzens (23) angebracht ist, und einen zweiten Auslassverbinder (24), an dem ein Ende (25A) eines zweiten Ausatmungsrohrstutzens (25) befestigt ist, umfasst.

5. Befeuchtungspatrone (10) nach Anspruch 1, **dadurch gekennzeichnet, dass** die Heizeinrichtung (12) durch Kontakt mit einer Heizplatte (60) eines Befeuchters (70) beheizt wird, wobei die Heizplatte (60) ihrerseits mittels mehrerer elektrischer Widerstände beheizt wird.

6. Befeuchtungspatrone (10) nach Anspruch 1, **dadurch gekennzeichnet, dass** der Befeuchter (70) einen Kasten (14) aufweist, der seine funktionalen Elemente wie etwa elektronische Schaltungen, die die elektrischen Widerstände in der Heizplatte (60) steuern/einstellen, und wenigstens einen Thermostaten enthält.

7. Befeuchtungspatrone (10) nach Anspruch 6, **dadurch gekennzeichnet, dass** der Befeuchter (70) ferner manuelle Regulierungsvorrichtungen (15), mittels derer eine Bedienungsperson den Heizzyklus des in der Kammer (13) enthaltenen Wassers im Voraus einstellen kann, umfasst.

8. Beatmungssystem (100) für einen Patienten (PZ); wobei das System (100) Folgendes umfasst:
- eine Beatmungseinrichtung (VNT);
- einen Beatmungskreis (50) für wenigstens ein Beatmungsgas von und zu dem Patienten (PZ);
**gekennzeichnet durch** wenigstens eine Befeuchtungspatrone (10) nach einem der Ansprüche 1 bis 7, wobei die Befeuchtungspatrone (10) und ein Sammler (20) für kondensiertes Wasser physikalisch ineinander integriert sind.

## Revendications

1. Cartouche (10) d'humidification comportant :
une chambre (13) de confinement et de chauffage d'un liquide susceptible de s'évaporer ; et
un élément chauffant (12) servant à chauffer le liquide contenu dans ladite chambre (13) ;
ladite cartouche (10) comportant un collecteur (20) d'eau condensée intégré dans ladite chambre (13),
**caractérisé en ce que** ledit collecteur (20) d'eau condensée comporte un corps principal (26), divisé en hauteur par une cloison filtrante microporeuse (27) et
fermé à sa partie inférieure par un clapet anti-retour (28).

2. Cartouche (10) d'humidification selon la revendication 1, **caractérisée en ce que** sur la paroi de ladite chambre (13) sont pratiquées une ouverture (16) d'entrée et une ouverture (17) de sortie, respectivement, d'un premier conduit (18) d'inhalation et d'un deuxième conduit (19) d'inhalation, et une ouverture (21) destinée à loger ledit collecteur (20) d'eau condensée.

3. Cartouche (10) d'humidification selon l'une des revendications 1 et 2, **caractérisée en ce que** le corps principal (26) est divisé par la cloison filtrante microporeuse (27) dans un espace supérieur (26A), défini par une paroi (29) de tête et par la cloison filtrante microporeuse (27), et un espace inférieur (26B) lui-même défini par la cloison filtrante microporeuse (27) et par le clapet (28).

4. Cartouche (10) d'humidification selon la revendication 3, **caractérisée en ce que** ledit collecteur (20) d'eau condensée comporte un premier connecteur (22) d'entrée sur lequel est montée une extrémité (23A) d'un premier conduit (23) d'exhalation, et un deuxième connecteur (24) de sortie sur lequel est montée une extrémité (25A) d'un deuxième conduit (25) d'exhalation.

5. Cartouche (10) d'humidification selon la revendication 1, **caractérisée en ce que** ledit élément chauffant (12) est chauffé par contact avec une plaque chauffante (60) d'un humidificateur (70), la plaque chauffante (60) étant elle-même chauffée au moyen d'une pluralité de résistances électriques.

6. Cartouche (10) d'humidification selon la revendication 1, **caractérisée en ce que** ledit humidificateur (70) comporte un carter (14) contenant ses éléments fonctionnels, tels que des circuits électroniques commandant / réglant les résistances électriques de la plaque chauffante (60), et au moins un thermostat.

7. Cartouche (10) d'humidification selon la revendication 6, **caractérisée en ce que** ledit humidificateur (70) comporte en outre des dispositifs (15) à régulation manuelle au moyen desquels un opérateur peut prérégler le cycle de chauffage de l'eau contenue dans ladite chambre (13).

8. Système de ventilation (100) destiné à un patient (PZ) ; ledit système (100) comportant
- un respirateur (VNT) ;
- un circuit (50) de ventilation d'au moins un gaz de ventilation en provenance du patient (PZ) et vers celui-ci ;
**caractérisé par** au moins une cartouche (10) d'humidification selon l'une quelconque des revendications 1 à 7, ladite cartouche (10) d'humidification et un collecteur (20) d'eau condensée étant physiquement intégrés l'un à l'autre.
